# EUROPEAN PATENT APPLICATION

(11) **EP 2 226 075 A1**
(43) Date of publication of application: **08.09.2010**
(21) Application number: 08852570.4
(22) Date of filing: 14.11.2008
(51) Int. Cl.: A61K 31/80, A61K 31/715, A61K 31/765, A61P 41/00, A61P 43/00

(54) **MATERIAL FOR PREVENTING TISSUE ADHESION AND MATERIAL FOR PREVENTING JOINT CONTRACTURE**

(30) Priority: 22.11.2007 JP 2007303389
(71) Applicant: The University of Tokyo, Bunkyo-Ku Tokyo 113-8654 (JP); KAKEN PHARMACEUTICAL CO., LTD., Bunkyo-ku Tokyo 113-8650 (JP)
(72) Inventor: MORO, Toru, Tokyo 113-8654 (JP); NAKAMURA, Kozo, Tokyo 113-8654 (JP); KAWAGUCHI, Hiroshi, Tokyo 113-8654 (JP); ISHIYAMA, Noriyuki, Tokyo 113-8654 (JP); ISHIHARA, Kazuhiko, Tokyo 113-8654 (JP); KONNO, Tomohiro, Tokyo 113-8654 (JP); OHYAMA, Tadashi, kyoto-shi Kyoto 607-8042 (JP); YOSHIKAWA, Mizuna, kyoto-shi Kyoto 607-8042 (JP)
(74) Representative: Denison, Christopher Marcus
(86) International application number: PCT/JP2008/071168
(87) International publication number: WO 2009/066746

(57) **Abstract**

The present invention provides a tissue adhesion prevention material preparable at an affected area at the time of surgical procedure by producing a three-dimensional polymeric structure having a flexible structure and high solute permeability in a medium comprising water as the main component under mild conditions appropriate for body tissue components (i.e., at ordinary temperature and pressure) without conducting a chemical reaction or employing a physical procedure such as heating or light or radiation irradiation. This makes it possible to provide a tissue adhesion prevention material and a joint contracture prevention materials, which can effectively prevent postoperative adhesion of a tissue in the affected area to the surrounding tissue and contracture of the movable part of a joint.

The tissue adhesion prevention material and/or the joint contracture prevention material of the present invention comprise, as the main component, a composition comprising a compound having a polyvalent hydroxyl group and a polymer containing phosphorylcholine groups and phenylboronic acid groups.

## Description

### TECHNICAL FIELD

The present invention relates to a biocompatible polymeric composite capable of preventing adhesion of a body tissue after surgical procedure to the surrounding tissue during the healing process and a tissue adhesion prevention material and a joint contracture prevention material, which consist of a three-dimensional crosslinked matrix thereof.

### BACKGROUND ART

In general, adhesion around joints (bones/muscles/ligaments) and nerves and adhesion of tendons generated after injury or surgery may cause joint movement disorder or nerve perceptual disorder, and this significantly interferes with social rehabilitation and daily living. For the purpose of repair and healing of such a damaged tissue, fixation is required for a certain period, and adhesion of the damaged tissue to the surrounding tissue almost always occurs. Such adhesion becomes a serious complication and a lot of time and effort is required for recovery of the function of a joint or nerve. In addition, further surgery may be required, or an irreversible disorder may be caused.

So far, various methods for adhesion prevention and prevention materials have been developed or tried (Tendon Adhesion Prevention Method, J Jpn Soc Surg Hand, Vol. 5(5), pp. 1016-1019, 1988; Prevention of restrictive adhesions with expanded polytetrafluoroethylene diffusible membrane following flexor tendon repair: an experimental study in rabbit, J Hand Surg, Vol. 23A, pp. 658-664, 1998; Experimental study on the prevention of tendon adhesion with hyaluronic acid-cinnamic acid film, J Jpn Soc Surg Hand, Vol. 16(6), pp. 876-886, 2000).

Further, for the purpose of adhesion prevention, not only the improvement of surgical techniques and materials, but also administration of an agent, early-stage exercise therapy, insertion of an adhesion prevention material into a damaged/surgery site and the like have been attempted. However, administration of an agent has not become widespread because of the problems of increased susceptibility to infection and toxicity to living bodies such as liver disorder. Further, early-stage exercise therapy has the risk of refracture or incomplete healing of a fracture site or second rupture of a nerve or tendon, and adaptation thereof to children and elderly persons is difficult. For the above-described reasons, it is not an effective solution.

So far, adhesion prevention materials made of synthetic polymeric materials have been developed. However, since the materials have no permeability with respect to liquid factors such as bioactive protein that promotes the growth of tissue, there are remaining problems that curing of a damaged tissue is adversely affected, that a foreign-body reaction is caused, and that adhesion is caused at the time of further surgery for removal.

On the other hand, in the case of adhesion prevention materials made of bioabsorbable materials, there are problems that a certain level of adhesion is difficult to avoid since cell infiltration is accompanied in the process of absorption, and that it is difficult to control the absorption speed *in vivo.*

In addition, there are problems regarding clinical therapy that it is difficult to handle such a material because of lack of flexibility, and that it is difficult to fix an adhesion prevention material to a target site. No adhesion prevention material, which can be easily handled in clinical practice, and which is highly effective in preventing adhesion of tissue, has been obtained yet.

### DISCLOSURE OF THE INVENTION

Therefore, it has been desired to develop a tissue adhesion prevention material by producing a three-dimensional polymeric structure having a flexible structure and high solute permeability in a medium comprising water as the main component under mild conditions appropriate for body tissue components (i.e., at ordinary temperatures and pressures) without conducting a chemical reaction or employing a physical procedure such as heating or light or radiation irradiation and by utilizing the structure. In addition, it has been desired to develop a tissue adhesion prevention material, which is safe even when indwelled *in vivo,* and which can be conveniently operated.

The present inventor diligently made researches in order to solve the above-described problems, and found that a reversible covalent bond is generated by a polymer having both phosphorylcholine groups and phenylboronic acid groups and a compound having a polyvalent hydroxyl group at ordinary temperature under ordinary pressure in a water-based solvent in a very short period of time to form water-insoluble three-dimensional crosslinked matrices. Moreover, it was found that the three-dimensional crosslinked matrices can be permeated not only by a low-molecular substance such as an agent but also by a protein having a relatively high molecular weight. Furthermore, it was found that the mesh size of the three-dimensional structure can be controlled by the concentration of the polymer to avoid cell infiltration, and that adhesion of cell or tissue to the polymeric crosslinked matrices themselves does not occur. Biocompatibility, extracorporeal elimination property, etc., which are newly desired for a tissue adhesion prevention material and a joint contracture prevention material, were combined with the above-described properties, and thus the present invention was achieved.

Specifically, the present invention is as follows:
(1) A tissue adhesion and/or joint contracture prevention materials, which comprises, as the main component, a composition comprising a compound having a polyvalent hydroxyl group and a polymer containing a phosphorylcholine group and a phenylboronic acid group.
(2) A tissue adhesion and/or joint contracture prevention material, which consists of three-dimensional crosslinked matrices formed by a composition comprising a compound having a polyvalent hydroxyl group and a polymer containing a phosphorylcholine group and a phenylboronic acid group.

Examples of the tissue adhesion and/or joint contracture prevention material of the present invention include those in which the compound having a polyvalent hydroxyl group is a polymer.

Examples of the tissue adhesion and/or joint contracture prevention material of the present invention include those in which the polymer containing a phosphorylcholine group and a phenylboronic acid group is represented by the following general formula (1): 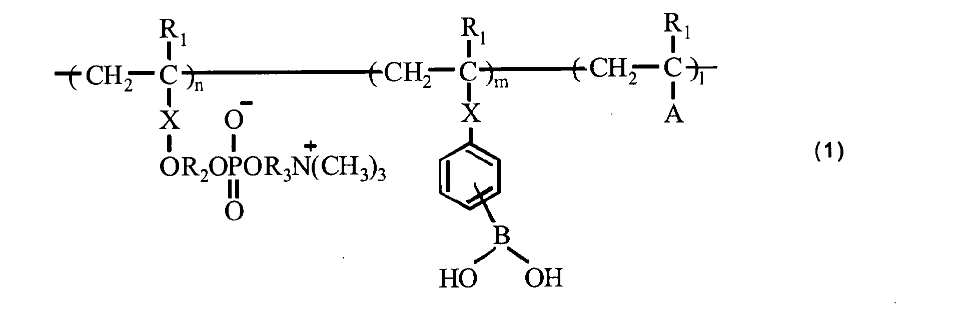 wherein: R₁ represents a hydrogen atom, a methyl group or an ethyl group; R₂ represents an alkyl group having 2 to 12 carbon atoms or an oxyethylene group; R₃ represents an alkyl group having 2 to 4 carbon atoms; X represents a single bond, a substituted or unsubstituted phenyl group, or a group represented by -C(O)-, -C(O)O-, -O-, -C(O)NH-or -S-; A represents a hydrogen atom, a halogen atom or any organic substituent; and n, m and 1 respectively represent 0.01 to 0.99, 0.01 to 0.99, and 0 to 0.98 (with proviso that the sum of n, m and 1 is 1.00).

Further, examples of the tissue adhesion and/or joint contracture prevention material of the present invention include those in which the compound having a polyvalent hydroxyl group is at least one selected from the group consisting of natural saccharides, synthetic saccharides and organic alcohols, and further include those in which the compound having a polyvalent hydroxyl group is at least one selected from the group consisting of polysaccharides and synthetic polymeric alcohols.

### BRIEF DESCRIPTION OF THE DRAWINGS

FIG. 1 shows the dissociation rate of a tissue adhesion prevention material consisting of the three-dimensional crosslinked composition of the present invention.
FIG. 2(A) shows a diffusion chamber used for evaluation of the dissociation rate of a gel-like composition in an *in vivo* model, and FIG. 2(B) shows the chamber being subcutaneously implanted in a rat. The chamber was removed 1 to 2 weeks after the implantation.
FIG. 3 shows macroscopic findings of the experiment shown in Example 16.
FIG. 4 shows SEM findings of the experiment shown in Example 16.
FIG. 5 shows adhesiveness of cells to the surface of a tissue adhesion prevention material (Example 6) consisting of the three-dimensional crosslinked composition of the present invention.
FIG. 6 shows a rat Achilles tendon 3 weeks after the suture (control group).
FIG. 7 shows rat Achilles tendons 3 weeks after the suture to which the BV gels obtained in Examples 9, 7 and 6 were applied.
FIG. 8 shows results of evaluation regarding the degree of adhesion of tendon based on the number of fibrous adhesions around the repaired tendon that required sharp dissection.
FIG. 9 shows results of evaluation regarding the degree of healing of tendon based on the maximal tensile strength represented the breaking strength at the repair site of a rat Achilles tendon.
FIG. 10 shows a rabbit FDP tendon 3 weeks after the suture (control group).
FIG. 11 shows a rabbit FDP tendon 3 weeks after the suture (when the BV gel of Example 7 was applied).
FIG. 12 shows results of evaluation regarding the degree of adhesion of tendon based on the number of fibrous adhesions around the repaired tendon that required sharp dissection.
FIG. 13 shows results of evaluation regarding the degree of healing based on the maximal tensile strength represented the breaking strength at the repair site of a rabbit FDP tendon.
FIG. 14 shows results of evaluation regarding the degree of adhesion of rat Achilles tendons to which the BV gels obtained in Examples 34, 35, 36, 37 and 38 were applied based on the number of fibrous adhesions around the repaired Achilles tendons that required sharp dissection.
FIG. 15 shows results of evaluation regarding the degree of adhesion of rat Achilles tendons to which the BV gels obtained in Examples 34, 35, 36, 37 and 38 were applied based on the ratio of the portion (range) adhered to the surrounding tissue in the circumference of Achilles tendon (360°), i.e., the adhesion rate (%).

### BEST MODE FOR CARRYING OUT THE INVENTION

Hereinafter, the present invention will be described in detail, but the scope of the present invention is not limited to the description. In addition to the following examples, the present invention can be suitably changed and then practiced within a range in which the effects of the present invention are not reduced.

Note that the entire specification of Japanese Patent Application No. 2007-303389, to which priority is claimed by the present application, is incorporated herein. In addition, all the publications such as prior art documents, laid-open publications, patents and other patent documents cited herein are incorporated herein by reference.

### 1. Method for producing the polymer (PMBV) containing a phosphorylcholine group and a phenylboronic acid group

The polymer to be used in the present invention can be produced by mixing a monomer containing a phosphorylcholine group and a monomer containing a phenylboronic acid group together in the solution state and subjecting the mixture to a radical polymerization reaction in the presence of a radical generation agent, and is a polymeric compound (PMBV) containing both phosphorylcholine groups and phenylboronic acid groups (simultaneously) (hereinafter also referred to as "the polymer of the present invention"). Note that a third monomer may be suitably added to adjust properties of a polymer to be produced. The polymer (PMBV) containing phosphorylcholine groups and phenylboronic acid groups has a structure represented by the following general formula (1): 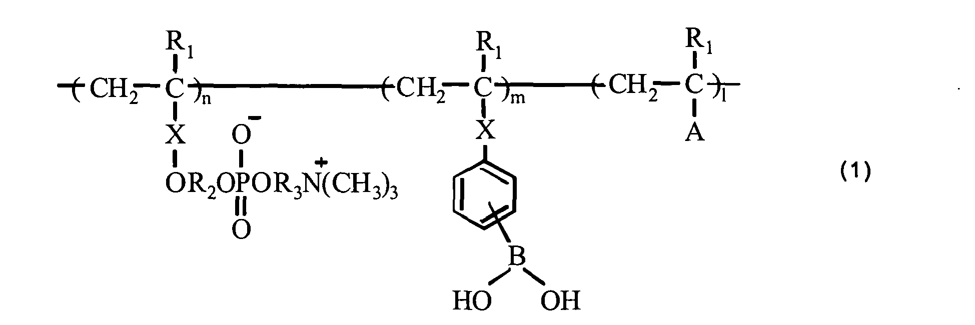 wherein: R₁ represents a hydrogen atom, a methyl group or an ethyl group; R₂ represents an alkyl group having 2 to 12 carbon atoms or an oxyethylene group; R₃ represents an alkyl group having 2 to 4 carbon atoms; X represents a single bond, a substituted or unsubstituted phenyl group, or a group represented by -C(O)-, -C(O)O-, -O-, -C(O)NH-or -S-; A represents a hydrogen atom, a halogen atom or any organic substituent; and n, m and 1 respectively represent 0.01 to 0.99, 0.01 to 0.99, and 0 to 0.98 (with proviso that the sum of n, m and 1 is 1.00).

The monomer having a phosphorylcholine group can be selected from compounds having a carbon-carbon double bond such as a vinyl group, allyl group, etc. as a polymerizable group and having a phosphorylcholine group in the same molecule.

Examples thereof include 2-methacryloyloxyethyl phosphorylcholine, 2-(meth)acryloyloxyethyl-2'-(trimethylammonio)ethyl phosphate, 3-(meth)acryloyloxypropyl-2'-(trimethylammonio)ethyl phosphate, 4-(meth)acryloyloxybutyl-2'-(trimethylammonio)ethyl phosphate, 5-(meth)acryloyloxypentyl-2'-(trimethylammonio)ethyl phosphate, 6-(meth)acryloyloxyhexyl-2'-(trimethylammonio)ethyl phosphate, 2-(meth)acryloyloxypropyl-2'-(trimethylammonio)ethyl phosphate, 2-(meth)acryloyloxybutyl-2'-(trimethylammonio)ethyl phosphate, 2-(meth)acryloyloxypentyl-2'-(trimethylammonio)ethyl phosphate, 2-(meth)acryloyloxyhexyl-2'-(trimethylammonio)ethyl phosphate, 2-(meth)acryloyloxyethyl-3'-(trimethylammonio)propyl phosphate, 3-(meth)acryloyloxypropyl-3'-(trimethylammonio)propyl phosphate, 4-(meth)acryloyloxybutyl-3'-(trimethylammonio)propyl phosphate, 5-(meth)acryloyloxypentyl-3'-(trimethylammonio)propyl phosphate, 6-(meth)acryloyloxyhexyl-3'-(trimethylammonio)propyl phosphate, 3-(meth)acryloyloxypropyl-4'-(trimethylammonio)butyl phosphate, 4-(meth)acryloyloxybutyl-4'-(trimethylammonio)butyl phosphate, 5-(meth)acryloyloxypentyl-4'-(trimethylammonio)butyl phosphate, and 6-(meth)acryloyloxyhexyl-4'-(trimethylammonio)butyl phosphate. In particular, 2-methacryloyloxyethyl phosphorylcholine (hereinafter abbreviated as MPC) is preferred. In this regard, "(meth)acryl" means "methacryl and/or acryl".

The monomer having phenylboronic acid groups can be selected from compounds having a carbon-carbon double bond such as a vinyl group, allyl group, etc. as a polymerizable group and having phenylboronic acid groups in the same molecule.

Examples thereof include p-vinylphenylboronic acid, *m-*vinylphenylboronic acid, *p*-(meth)acryloyloxyphenylboronic acid, *m*-(meth)acryloyloxyphenylboronic acid, *p*-(meth)acrylamide phenylboronic acid, *m*-(meth)acrylamide phenylboronic acid, *p-*vinyloxyphenylboronic acid, *m*-vinyloxyphenylboronic acid, and vinyl urethane phenylboronic acid. However, in view of easiness of obtaining raw materials, *p-*vinylphenylboronic acid or *m*-vinylphenylboronic acid is desired.

The third monomer which can be added is used for the purpose of imparting hydrophobic property, charge property, and chemical bonding property with respect to equipments to the polymer of the present invention.

Examples thereof include: hydrophilic monomers such as (meth)acrylic acid, sodium (meth)acrylate, 2-hydroxyethyl (meth)acrylate, glycerol (meth)acrylate, N-vinyl pyrrolidone, acrylonitrile, (meth)acrylamide, polyethylene glycol mono(meth)acrylate, vinylbenzene sulfonic acid, and sodium vinylbenzenesulfonate; hydrophobic monomers such as methyl (meth)acrylate, ethyl (meth)acrylate, butyl (meth)acrylate, lauryl (meth)acrylate, dodecyl (meth)acrylate, stearyl (meth)acrylate, 2-ethylhexyl (meth)acrylate, styrene, and vinyl acetate; monomers having an alkyloxysilane group such as (3-methacryloyloxypropyl)trimethoxysilane, (3-methacryloyloxypropyl)triethoxysilane, (3-methacryloyloxypropyl)methyldimethoxysilane, and trimethoxyvinylsilane; monomers having a siloxane group; monomers having a glycyl group such as glycidyl methacrylate, monomers having an amino group such as allylamine, aminoethyl (meth)acrylate, and 2-methylallylamine; and monomers having a group such as carboxyl, hydroxyl, aldehyde, thiol, halogen, methoxy, epoxy, succinimide, and maleimide. In particular, butyl (meth)acrylate is preferred. The above-described substances can be used solely or in combination.

At the time of polymerization reaction, monomers are preferably in the state of homogeneous solution. When using solid monomers, a solvent which homogeneously dissolves the monomers may be added. Moreover, using a solvent which can dissolve a polymer produced is preferred for obtaining a polymer having a stable structure. One type of solvent may be used, or two or more types of solvents may be mixed together to be used as a mixed solvent.

The radical generation agent is not limited as long as it is dissolved in a monomer mixture and decomposed at a reaction temperature of 30°C to 90°C to generate radicals. However, in terms of safety and stability, aliphatic azo compounds such as azobisisobutyronitrile and 4,4'-azobis(4-cyanopentanoic acid) and peroxides such as benzoyl peroxide, succinic peroxide and t-butylperoxyneodecanoate are preferred.

Moreover, the molecular structure and molecular weight may be controlled utilizing an initiator for generating radicals by light irradiation, atom transfer living radical polymerization reaction, reversible addition-fragmentation chain transfer polymerization or the like.

The mole fraction composition of the monomer unit having a phosphorylcholine group in the polymer of the present invention can be controlled by the composition in the monomer mixture solution, and the range thereof is 0.01 to 0.99, preferably 0.05 to 0.80, and more preferably 0.30 to 0.70.

The above-described ranges are preferred in order to maintain the polymer's good solubility in an aqueous medium and good biocompatibility.

The mole fraction composition of the monomer unit having a phenylboronic acid group in the polymer of the present invention can be controlled by the composition in the monomer mixture solution, and the range thereof is 0.01 to 0.99, preferably 0.03 to 0.50, and more preferably 0.05 to 0.20.

The above-described ranges are preferred in order to maintain good reactivity with a compound having a polyvalent hydroxyl group, good strength of three-dimensional crosslinked matrices to be produced and good solubility of the polymer in an aqueous medium.

The composition of the third monomer that can be added is represented by the difference between the entire monomer, and the monomer having a phosphorylcholine group and the monomer having a phenylboronic acid group.

When performing measurement using gel permeation chromatography, the molecular weight of the polymer of the present invention can be converted based on polyethylene oxide as a reference substance, and the range thereof is 1,000 to 10,000,000, preferably 2,000 to 1,000,000, and more preferably 3,000 to 1,000,000. Moreover, in terms of the ability to produce three-dimensional crosslinked matrices, solubility in an aqueous medium, discharge from the body, etc., it is desired that the range is 4,000 to 100,000.

### 2. Method for producing the compound having a polyvalent hydroxyl group

It is preferred that the compound having a polyvalent hydroxyl group is dissolved in a water-based medium and becomes a homogeneous solution. Specific examples thereof include natural saccharides, synthetic saccharides, organic alcohols and polymers. Preferred examples of compounds having a polyvalent hydroxyl group included in the natural saccharides include: monosaccharides such as glucose and glucosamine; disaccharides such as maltose and lactose; polysaccharides such as amylose, amylopectin, chitin, hyaluronic acid, celluloses and derivatives thereof; and the like. Preferred examples of compounds having a polyvalent hydroxyl group included in the synthetic saccharides include pullulan and dextran. Preferred examples of compounds having a polyvalent hydroxyl group included in the organic alcohols include low-molecular polyhydric alcohols such as synthetic diols and triols. Preferred examples of compounds having a polyvalent hydroxyl group included in the polymers include polyvinyl alcohol, poly(2-hydroxyethyl (meth)acrylate), poly(2,3-dihydroxyethyl (meth)acrylate), poly((meth)acrylic acid glycoside) and the like, and water-soluble polymeric alcohols comprising, as one component, a monomer unit constituting the above-described polymers.

Among them, polysaccharides and polymeric alcohols are preferably selected as the compound having a polyvalent hydroxyl group since three-dimensional crosslinked matrices having a stable structure can be produced using them in a short period of time. In particular, polyvinyl alcohol is preferred. When performing measurement using gel permeation chromatography, the molecular weight in this case can be converted based on polyethylene oxide as a reference substance, and the range thereof is 1,000 to 10,000,000, and preferably 2,000 to 1,000,000. Moreover, in terms of the ability to produce three-dimensional crosslinked matrices and the solubility in an aqueous medium, it is desired that the range is 3,000 to 600,000.

### 3. Method for producing the three-dimensional crosslinked matrix

The three-dimensional crosslinked matrices can be produced by mixing a water-based solution comprising the compound having a polyvalent hydroxyl group with a water-based solution comprising the polymer containing a phosphorylcholine group and phenylboronic acid groups. As a medium for preparing a water-based solution, pure water, a buffer solution, and an aqueous solution containing an organic solvent in an amount of 30% or less may be used. In order to appropriately maintain safety with respect to body tissue, the content of the organic solvent is preferably 30% or less.

The compound having a polyvalent hydroxyl group and the polymer containing phosphorylcholine groups and phenylboronic acid groups simultaneously can be used at a concentration at which they can be dissolved in an aqueous solution prepared. However, in view of viscosity and stability of the net-like structure of three-dimensional crosslinked matrices obtained, each concentration is preferably 0.5 to 20 wt%, and more preferably 0.7 to 10 wt%.

The temperature for obtaining the three-dimensional crosslinked matrices is 4 to 40°C. In particular, when used at the time of surgical operation, also in view of operability, the temperature is preferably 22 to 39°C, i.e., about room temperature to body temperature.

### 4. Adhesion prevention material and joint contracture prevention material

In the present invention, a composition comprising the compound having a polyvalent hydroxyl group and the polymer of the present invention can be used as a tissue adhesion prevention material, or a joint contracture prevention material, or a material for preventing both tissue adhesion and joint contracture.

As used herein, "adhesion" means that organs/tissues, which should be separated from each other are connected/fused together by fibrous tissue.

Further, "joint contracture" means that the joint motion is limited by adhesion of periarticular tissue.

As used herein, "prevention" means preventing symptoms from occurring, reducing the degree of symptom occurrence, suppressing progression of symptoms, etc.

As a method for using the adhesion and/or contracture prevention material of the present invention, for example, a composition (a three-dimensional crosslinked matrix) comprising the polymer of the present invention can be applied (e.g., adhered and coated) to a damaged body tissue (to a damaged site or around the sutured portion of the damaged site). Alternatively, a water-based solution comprising the compound having a polyvalent hydroxyl group may be mixed with a water-based solution comprising the polymer containing phosphorylcholine groups and phenylboronic acid groups at an affected area locally at the time of surgical procedure, thereby preparing three-dimensional crosslinked matrices for application. The amount of application may be suitably set depending on the degree of damage or with reference to working examples.

### EXAMPLES

Hereinafter, the present invention will be described in more detail based on working examples, but the present invention is not limited thereto.

### [Example 1]

### <Synthesis of polymer (PMBpV) containing a phosphorylcholine group and a phenylboronic acid group simultaneously>

53 g of 2-methacryloyloxyethyl phosphorylcholine (abbreviated as MPC) was weighed and put into a flask, and 300 mL of ethanol was added thereto. With stirring, the inside of the container was subjected to substitution with argon. Next, 4.4 g of p-vinylphenylboronic acid (abbreviated as p-VPB), 13 g of n-butyl methacrylate (abbreviated as BMA) and 0.49 g of 2,2'-azobisisobutyronitrile were added thereto, and it was stirred so that it became homogeneous. After the flask was plugged with an airtight stopper, it was heated to 60°C and stirred for 48 hours. The obtained solution was taken out therefrom, and the solution was added dropwise to 6000 mL of a mixed solution of diethylether/chloroform (8/2) to obtain a solid polymer. The yield was 50 g and 71%. This was dried under reduced pressure, thereby obtaining a polymer (PMBpV). Note that PMBpV means one type of the polymer of the present invention, PMBV, in which boronic asid groups in phenylboronic acid groups in the polymer are bound to the para position (the same applies to the following). This polymer was analyzed according to the IR analysis conditions for the aforementioned polymer. As a result, infrared absorption derived from a phenyl group was observed at 3,600 cm⁻¹, infrared absorption derived from ester bond was observed at 1,730 cm⁻¹, and infrared absorption derived from phosphorylcholine groups was observed at 1,200 to 1,100 cm⁻¹. According to the result of the NMR measurement, the composition of the monomer units in the polymer was as follows: MPC/p-VPB/BMA = 58/11/31 (mole %). The molecular weight was obtained utilizing gel permeation chromatography, and calculation was made utilizing polyethylene oxide as a reference substance. As a result, the number average molecular weight was 39,000.

### [Example 2]

### <Synthesis of polymer (PMBmV) containing a phosphorylcholine group and a phenylboronic acid group simultaneously>

5.3 g of MPC was weighed and put into a test tube, and 25 mL of ethanol was added thereto. With stirring, the inside of the container was subjected to substitution with nitrogen. Next, 0.44 g of m*-*vinylphenylboronic acid (abbreviated as m-VPB), 1.3 g of BMA and 0.049 g of 2,2'-azobisisobutyronitrile were added thereto, and 5 g of tetrahydrofuran (THF) was further added thereto, and it was stirred under nitrogen atmosphere so that it became homogeneous. After that, the test tube was sealed. It was heated to 60°C and stirred for 24 hours. The obtained solution was taken out therefrom, and the solution was added dropwise to 500 mL of a mixed solution of diethylether/chloroform (9/1) to obtain a solid polymer. The yield was 4.2 g and 60 %. This was dried under reduced pressure, thereby obtaining a polymer (PMBmV). Note that PMBmV means one type of the polymer of the present invention, PMBV, in which boronic acid groups in phenylboronic acid groups in the polymer are bound to the meta position (the same applies to the following). This polymer was analyzed according to the IR analysis conditions for the aforementioned polymer. As a result, infrared absorption derived from a phenyl group was observed at 3,600 cm⁻¹, infrared absorption derived from ester bond was observed at 1,730 cm⁻¹, and infrared absorption derived from a phosphorylcholine group was observed at 1,200 to 1,100 cm⁻¹. According to the result of the NMR measurement, the composition of the monomer units in the polymer was as follows: MPC/m-VPB/BMA = 60/13/27 (mole %). The molecular weight was obtained utilizing gel permeation chromatography, and calculation was made utilizing polyethylene oxide as a reference substance. As a result, the number average molecular weight was 52,000.

### [Example 3]

### <Synthesis of polymer (PMBpV) containing a phosphorylcholine group and a phenylboronic acid group simultaneously>

1.69 g of 2-acryloyloxyethyl phosphorylcholine (abbreviated as APC) was weighed and put into a test tube, and 20 mL of ethanol was added thereto. With stirring, the inside of the container was subjected to substitution with nitrogen. Next, 148 mg of p-VPB, 426 mg of BMA and 23.4 mg of benzoyl peroxide were added thereto, and 1.90 g of N,N-dimethylformamide was further added thereto, and it was stirred under nitrogen atmosphere so that it became homogeneous. After that, the test tube was heat-sealed. It was heated to 70°C in an oil bath and stirred for 12 hours. The obtained solution was taken out therefrom, and the solution was added dropwise to 200 mL of a mixed solution of diethylether/chloroform (8/2) to obtain a solid polymer. The yield was 1.81 g and 80 %. This was dried under reduced pressure, thereby obtaining a polymer (PMBpV). This polymer was analyzed according to the IR analysis conditions for the aforementioned polymer. As a result, infrared absorption derived from a phenyl group was observed at 3,600 cm⁻¹, infrared absorption derived from ester bond was observed at 1,730 cm⁻¹, and infrared absorption derived from a phosphorylcholine group was observed at 1,200 to 1,100 cm⁻¹. According to the result of the NMR measurement, the composition of the monomer units in the polymer was as follows: APC/p-VPB/BMA = 70/9/21 (mole %). The molecular weight was obtained utilizing gel permeation chromatography, and calculation was made utilizing polyethylene oxide as a reference substance. As a result, the number average molecular weight was 64,000.

### [Example 4]

### <Synthesis of polymer (PMBmV) containing a phosphorylcholine group and a phenylboronic acid group simultaneously>

1.69 g of APC was weighed and put into a test tube, and 30 mL of ethanol was added thereto. With stirring, the inside of the container was subjected to substitution with nitrogen. Next, 148 mg of m-VPB, 426 mg of BMA and 23.4 mg of benzoyl peroxide were added thereto, and 1.90 g of N,N-dimethylformamide was further added thereto, and it was stirred under nitrogen atmosphere so that it became homogeneous. After that, the test tube was heat-sealed. It was heated to 65°C in an oil bath and stirred for 8 hours. The obtained solution was taken out therefrom, and the solution was added dropwise to 100 mL of a mixed solution of diethylether/chloroform (8/2) to obtain a solid polymer. The yield was 1.36 g and 60 %. This was dried under reduced pressure, thereby obtaining a polymer (PMBmV). This polymer was analyzed according to the IR analysis conditions for the aforementioned polymer. As a result, infrared absorption derived from a phenyl group was observed at 3,600 cm⁻¹, infrared absorption derived from ester bond was observed at 1,730 cm⁻¹, and infrared absorption derived from a phosphorylcholine group was observed at 1,200 to 1,100 cm⁻¹. According to the result of the NMR measurement, the composition of the monomer units in the polymer was as follows: APC/m-VPB/BMA = 60/13/27 (mole %). The molecular weight was obtained utilizing gel permeation chromatography, and calculation was made utilizing polyethylene oxide as a reference substance. As a result, the number average molecular weight was 47,000.

### [Example 5]

### <Synthesis of polymer (PMBmV) containing a phosphorylcholine group and a phenylboronic acid group simultaneously>

A polymer was obtained by the same operation as that of Example 1, except that 5.7 g of *m*-acrylamide phenylboronic acid (hereinafter abbreviated as APB) was used instead of *p*-VPB. The yield was 41.5 g and 58 %. This was dried under reduced pressure, thereby obtaining a polymer (PMBmV). This polymer was analyzed according to the IR analysis conditions for the aforementioned polymer. As a result, infrared absorption derived from a phenyl group was observed at 3,600 cm⁻¹, infrared absorption derived from ester bond was observed at 1,730 cm⁻¹, and infrared absorption derived from a phosphorylcholine group was observed at 1,200 to 1,100 cm⁻¹. According to the result of the NMR measurement, the composition of the monomer units in the polymer was as follows: MPC/APB/BMA = 61/19/20 (mole %). The molecular weight was obtained utilizing gel permeation chromatography, and calculation was made utilizing polyethylene oxide as a reference substance. As a result, the number average molecular weight was 81,000.

### [Examples 6-15]

### <Preparation of three-dimensional crosslinked matrx>

Each of the polymers obtained in Examples 1 to 5 was dissolved in water to prepare a polymer aqueous solution at a predetermined concentration. Meanwhile, polyvinyl alcohol (abbreviated as PVA) was dissolved in warm water to prepare an aqueous solution, and after that, it was diluted with water to a predetermined concentration. PVA having a polymerization degree of 900 to 1,100 (number average molecular weight: 44,000) (abbreviated as PVA1000) was used. By mixing them together at room temperature, three-dimensional crosslinked matrices were prepared. Table 1 shows the results of formation of three-dimensional crosslinked bodies at respective concentrations. It is understood from the results in Table 1 that the mixed solution, which is a liquid, became three-dimensional crosslinked matrices and that the solid (gel) state was achieved with the medium included. Further, a three-dimensional crosslinked matrices can be produced even when the polymer concentration and the mixed composition are changed. Here, as criteria for judgment of production of three-dimensional crosslinked bodies, the following sensory indexes were determined:
○: the whole liquid loses flow ability and three-dimensional crosslinked matrices of polymer are completely produced;
△: a part of liquid remains, and three-dimensional crosslinked matrices are partially produced;
×: the liquid state is maintained, and the production of three-dimensional crosslinked matrices are not observed.

**Table 1**

| Three-dimenitional crosslinked matrices | PMBV | Concentration ( % ) | P V A concentration ( % ) | Judgment |
|---|---|---|---|---|
| Example 6 | Example 1 | 5 | 5 | ○ |
| Example 7 | Example 1 | 5 | 2.5 | ○ |
| Example 8 | Example 1 | 1.25 | 5 | △ |
| Example 9 | Example 1 | 2.5 | 2.5 | ○ |
| Example 10 | Example 1 | 0.63 | 5 | × |
| Example 11 | Example 1 | 0.31 | 5 | × |
| Example 12 | Example 2 | 5 | 5 | ○ |
| Example 13 | Example 3 | 5 | 5 | ○ |
| Example 14 | Example 4 | 5 | 5 | ○ |
| Example 15 | Example 5 | 5 | 5 | ○ |

### [Example 16]

### <Observation of structure of a three-dimensional crosslinked matrix (abbreviated as BV gel)>

The three-dimensional crosslinked matrix obtained in Example 6 was freeze-dried to prepare an observation sample. The cross-sectional surface of the sample was observed using a scanning electron microscope, and it was found that the three-dimensional crosslinked matrix which was porous was produced and that the pore diameter thereof was about 1 µm.

### [Example 17]

### <Measurement of gel decomposition rate in phosphate buffer>

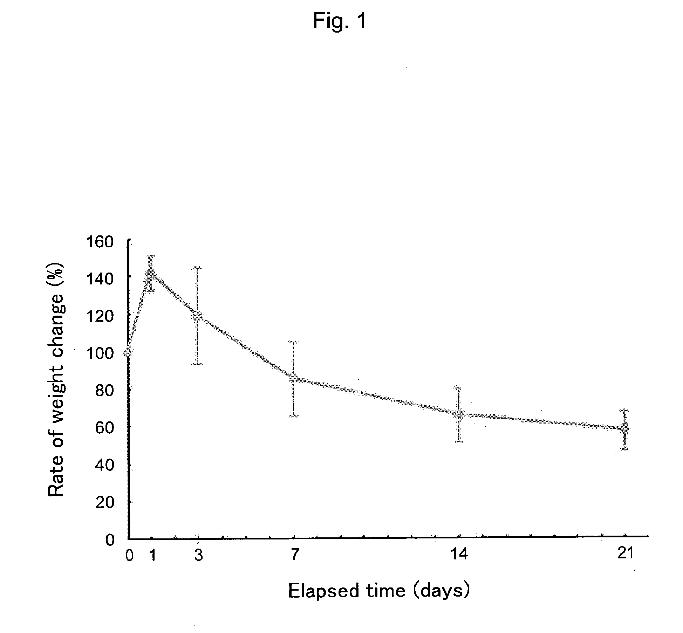

0.5 mL of 5% aqueous solution of PMBpV obtained in Example 1 was mixed with 0.5 mL of 5% aqueous solution of PVA to prepare a BV gel. Next, a silicone pack containing the BV gel was immersed in 30 mL of phosphate buffer, and the weight thereof was measured over time. The results are shown in Figure 1. The vertical axis of Figure 1 represents the ratio of the weight of the silicone pack after permeation of phosphate buffer to the weight before permeation. The decrease in the weight of the silicone pack means that the gel was decomposed to leak out to the outside of the pack, and dissociation and disappearance of the gel was observed in the biological environment.

### [Example 18]

### <Study on dissociation rate of three-dimensional crosslinked matrix in in vivo model>

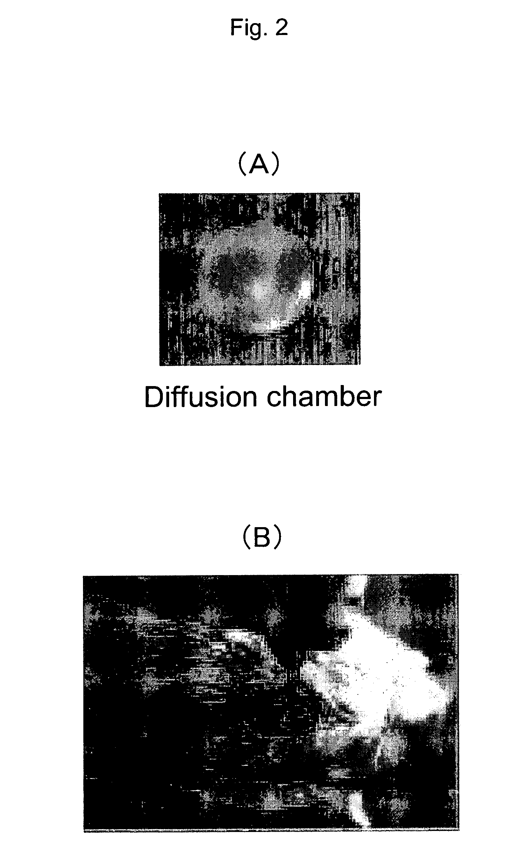

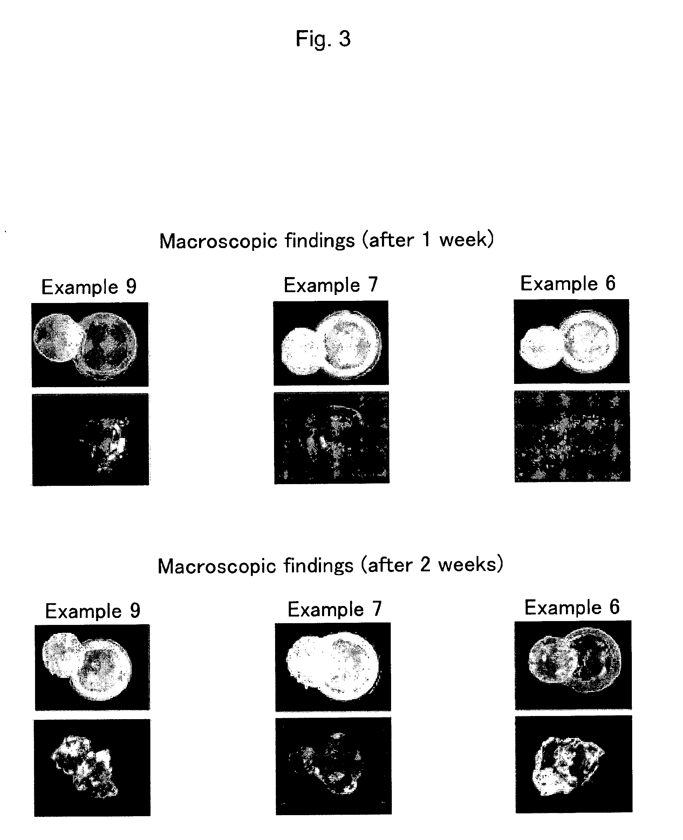

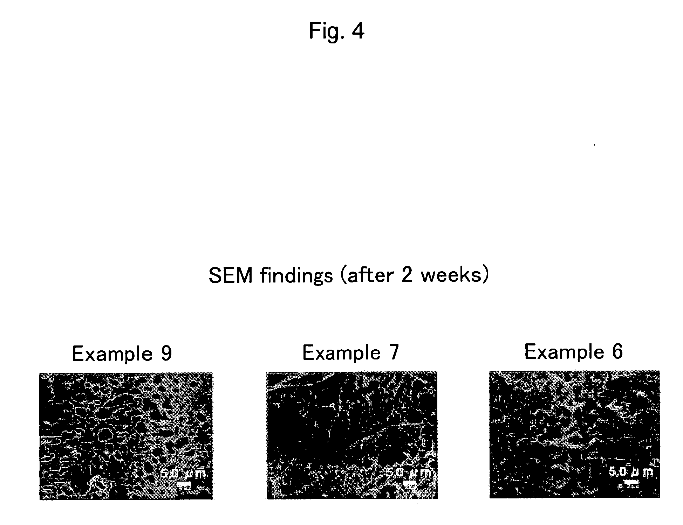

Diffusion chambers in which a cellulose film having the pore diameter of 0.22 µm was attached to the both surfaces of a plastic ring were prepared. BV gels of Examples 6, 7 and 9, which were prepared with the concentration of the polymer aqueous solution changed, were respectively put into the diffusion chambers. The chambers were subcutaneously implanted in a rat. After 1 or 2 weeks, the diffusion chambers were removed (Figure 2), and the gel dissociation rate was evaluated based on macroscopic findings and scanning electron microscopical (SEM) findings. The macroscopic findings are shown in Figure 3. One week after the implantation, there was no clear difference among the findings of the 3 types of gels, but 2 weeks after the implantation, the gels remained depending on each of the aqueous solution concentrations of PMBpV and PVA. Thus, the dissociation rate of the gel could be controlled by changing the aqueous solution concentration. The SEM findings are shown in Figure 4. It was shown that the three-dimensional net-like structure was maintained even 2 weeks after the implantation. The above-described results show that the BV gel can keep the properties of three-dimensional crosslinked matrices under the skin of a rat for at least 2 weeks, and that the dissociation rate can be controlled by the aqueous solution concentration.

### [Example 19]

### <Cellular adhesiveness>

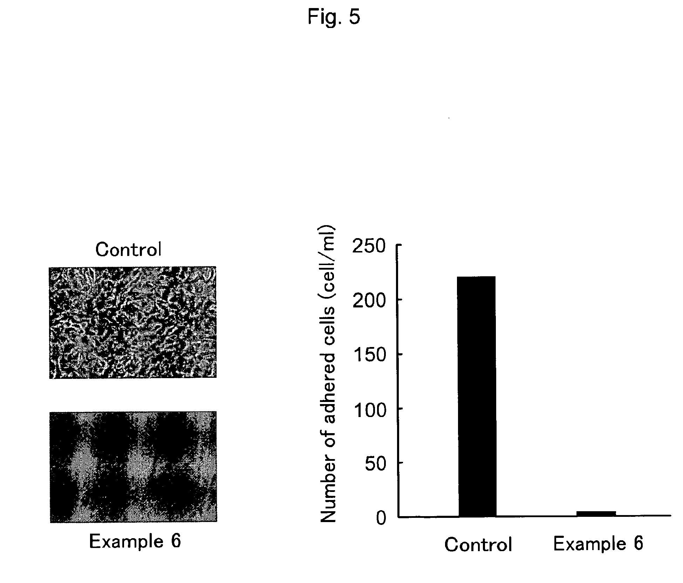

The culture surface of a cell culture dish was coated with the three-dimensional crosslinked BV gel of Example 6. Next, a mouse fibroblast-like cell line (NIH3T3) was cultured on the surface, and its time-dependent state of cell adhesion was compared to that of an uncoated dish (control group). The results obtained 36 hours after the culture are shown in Figure 5. In the case of the control group, cells were adhered and grown, whereas in the case of the dish coated with the BV gel, almost no cells were adhered and in a suspended state. Further, when the suspended cells were collected and cultured on an untreated dish, the cells were adhered to the dish and grown like the control group. Therefore, the influence of cytotoxicity due to contact with the BV gel was excluded. Thus, it became clear that the BV gel has the effect of suppressing cell adhesion and does not have cytotoxicity, and it was shown that the BV gel is effective as an adhesion prevention material.

### [Example 20]

### <Study on healing/adhesion in Achilles tendon-damaged rat model>

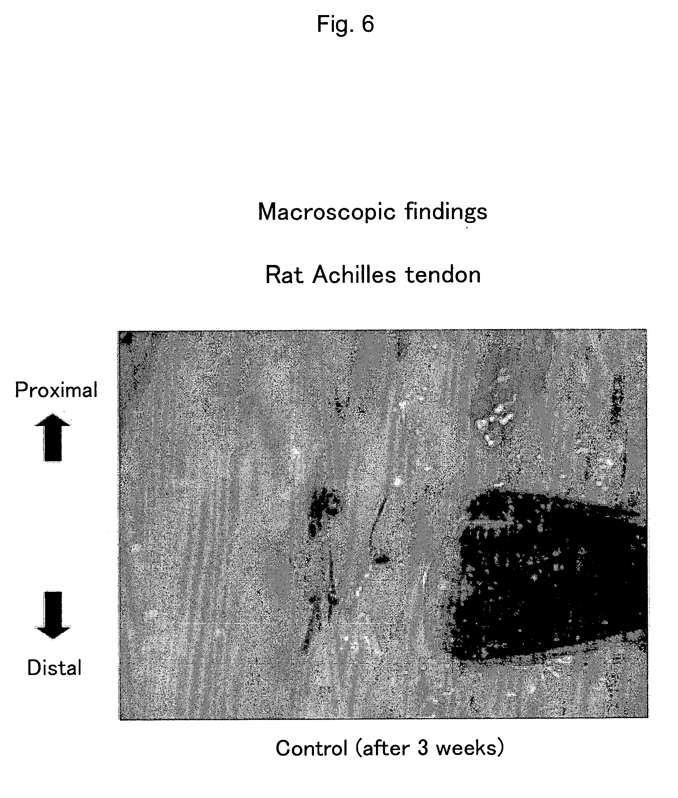

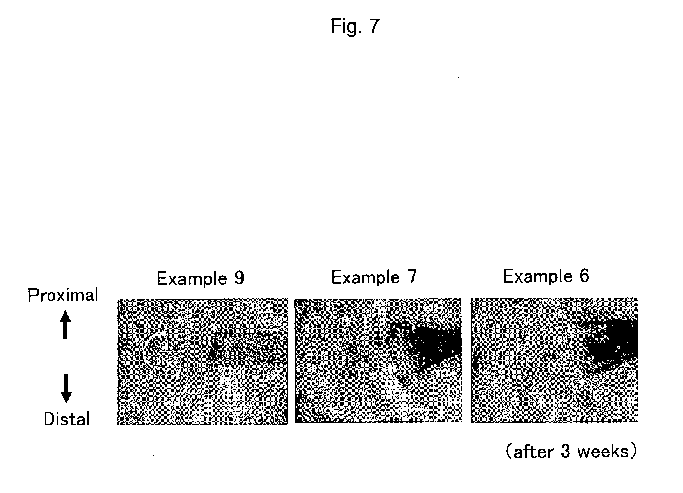

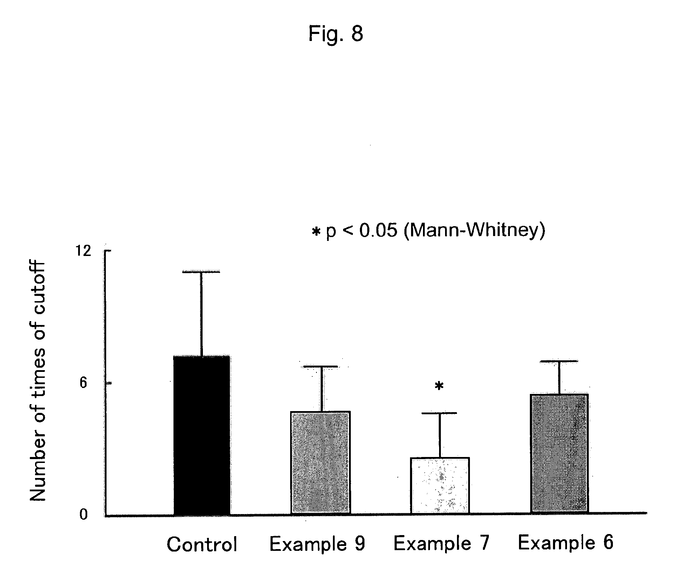

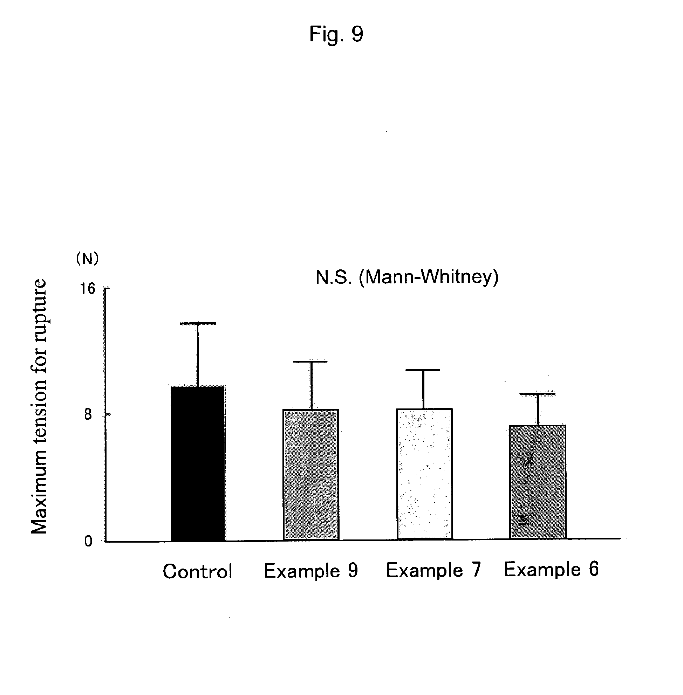

An Achilles tendon of the right foot of a rat was cut and sutured, and the BV gels of Examples 6, 7 and 9, which were prepared by changing the aqueous solution concentration of the polymer, were adhered to the area surrounding the sutured portion. After the wound was closed, the foot was externally fixed using a plaster cast. The wound was opened 3 weeks after the surgery, and the sutured portion was observed and the tendon was collected. Healing/adhesion was evaluated based on the macroscopic findings of the sutured portion and the dynamic findings of the tendon. Note that "healing" means that a wound portion/damaged site is cured, and that separated tissues are bound together. In the case of the control group 3 weeks after the suture (only saline, which is a solvent of the polymer aqueous solution, was used), adhesion to the surrounding area was obvious, and it was difficult to bluntly detach the adhesion (Figure 6). On the other hand, in the case of the BV gels 3 weeks after the suture, adhesion to the surrounding area was suppressed at each concentration (Figure 7). When the degree of adhesion of the tendon was evaluated based on the number of fibrous adhesions around the repaired tendon that required sharp dissection, it became clear that adhesion of the tendon was significantly suppressed, in particular, by the BV gel of Example 7 (Figure 8). When the degree of healing of the tendon was evaluated based on the maximal tensile strength required for rupturing the tendon, it was found that there was no significant difference between the control group and the BV gel group (Figure 9). According to the working example, it was shown that the BV gel does not inhibit healing of the tendon even if the aqueous solution concentration is changed. Further, it was shown that adhesion around the sutured portion is significantly suppressed particularly by the BV gel of Example 7.

### [Example 21]

### <Study on healing/adhesion in flexor digitorum profundus tendon-damaged rabbit model>

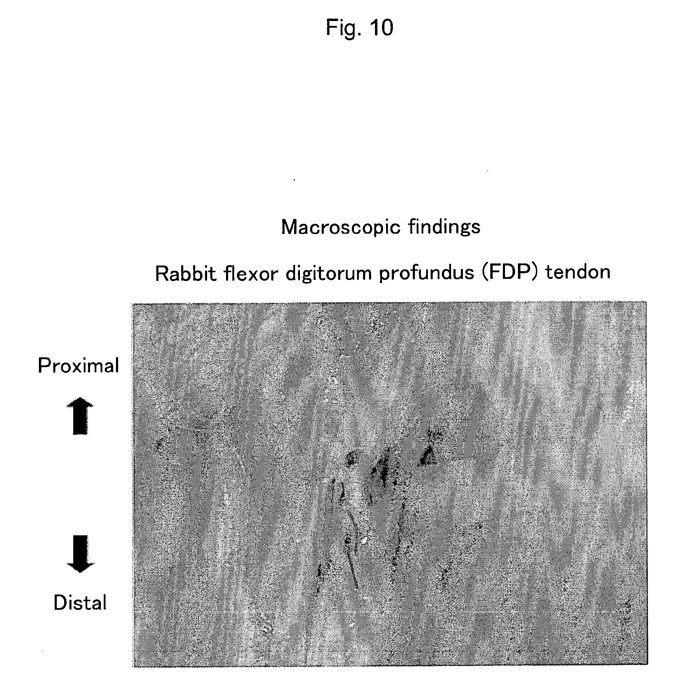

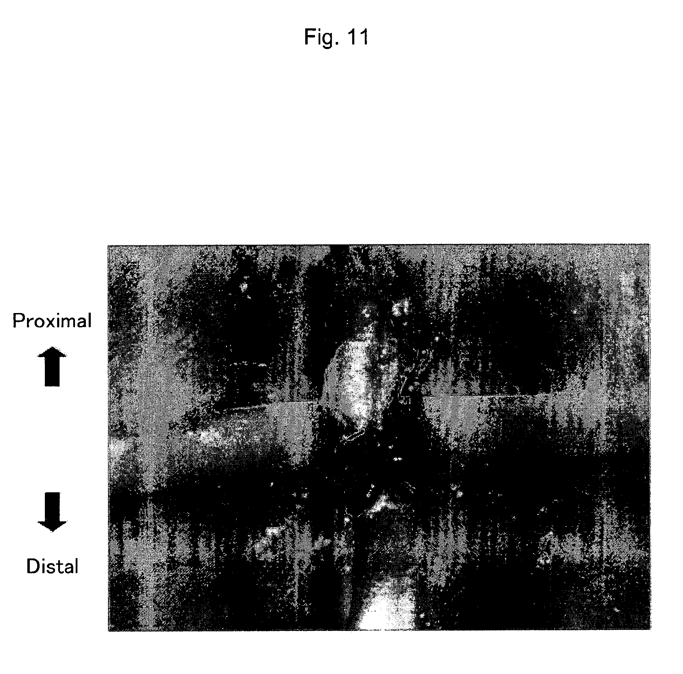

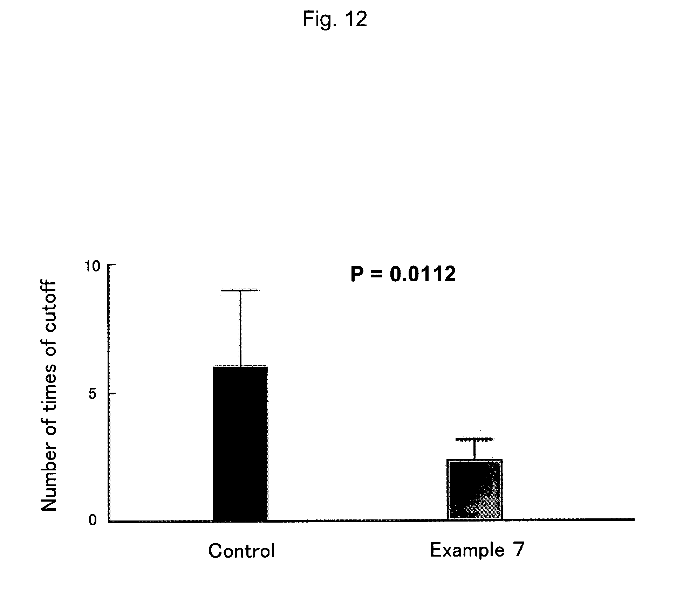

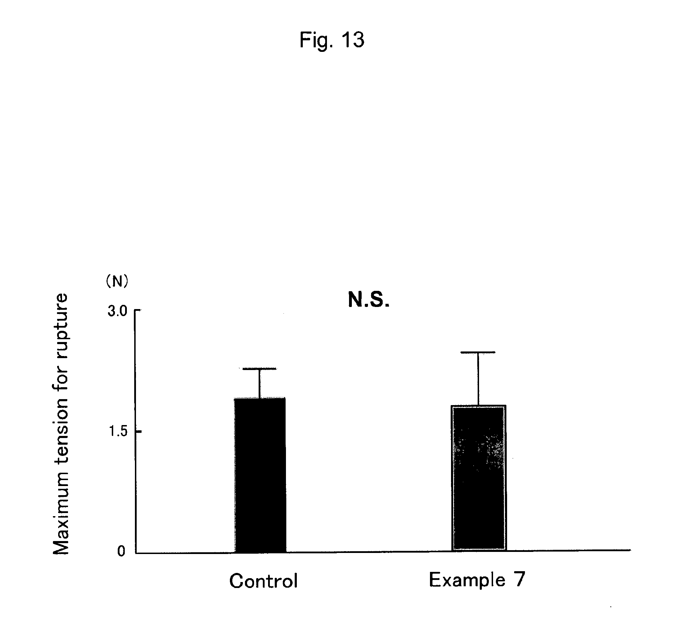

A flexor digitorum profundus (abbreviated as FDP) tendon of the third toe of the left front foot of a rabbit was cut and then sutured. Next, the BV gel of Example 7, which showed particularly high effect of adhesion prevention in Example 20, was adhered to the surrounding area of the sutured portion. After that, the wound was closed, and the foot was externally fixed using a plaster cast. The wound was opened 3 weeks after the surgery, and the sutured portion was macroscopically observed. After that, the tendon was collected. Healing/adhesion was evaluated based on the macroscopic findings of the sutured portion and the dynamic findings of the tendon. In the case of the control group 3 weeks after the suture (only saline was used), it was difficult to bluntly detach the adhesion, and a vessel tape could not be passed through to the back side of the tendon. Thus, adhesion to the surrounding area was obvious (Figure 10). On the other hand, in the case of the BV gel group 3 weeks after the suture, almost no adhesion to the surrounding area was observed. Even when only blunt detachment was performed, a vessel tape was easily passed through to the back side of the tendon (Figure 11). Next, when the degree of adhesion of the tendon was evaluated based on the number of fibrous adhesions around the repaired tendon that required sharp dissection, it was found that the BV gel group significantly suppressed the adhesion (Figure 12). Further, when the degree of healing of the tendon was evaluated based on the maximal tensile strength required for rupturing the tendon, it was found that there was no significant difference between the control group and the BV gel group (Figure 13). According to the working example, it was shown that the BV gel does not inhibit healing of the tendon and significantly suppresses adhesion of the surrounding area of the sutured portion.

### [Example 22]

### <Synthesis of polymer (PMBpV) containing a phosphorylcholine group and a phenylboronic acid group simultaneously>

4.4 g of MPC was weighed and put into a test tube, and 23 mL of ethanol was added thereto. With stirring, the inside of the container was subjected to substitution with argon. Next, 0.9 g of p-VPB, 1.3 g of BMA and 0.25 g of 2,2'-azobisisobutyronitrile were added thereto, and the mixture was stirred so that it became homogeneous. After that, the test tube was sealed. It was heated to 60°C and stirred for 4 hours. The obtained solution was taken out therefrom, and the solution was added dropwise to 500 mL of a mixed solution of diethylether/chloroform (8/2) to obtain a solid polymer. The yield was about 70%. This was dried under reduced pressure, thereby obtaining a polymer (PMBpV). This polymer was analyzed according to the IR analysis conditions for the aforementioned polymer. As a result, infrared absorption derived from a phenyl group was observed at 3,600 cm⁻¹, infrared absorption derived from ester bond was observed at 1,730 cm⁻¹, and infrared absorption derived from a phosphorylcholine group was observed at 1,200 to 1,100 cm⁻¹. According to the result of the NMR measurement, the composition of the monomer units in the polymer was as follows: MPC/p-VPB/BMA = 53/16/31 (mole %). The molecular weight was obtained utilizing gel permeation chromatography, and calculation was made utilizing polyethylene oxide as a reference substance. As a result, the number average molecular weight was 15,000.

### [Example 23]

### <Synthesis of polymer (PMBpV) containing a phosphorylcholine group and a phenylboronic acid group simultaneously>

A polymer was obtained by the same operation as that of Example 22, except that 3.7 g of t-butylperoxyneodecanoate was used instead of 2,2'-azobisisobutyronitrile. The yield was about 70%. This was dried under reduced pressure, thereby obtaining a polymer (PMBpV). This polymer was analyzed according to the IR analysis conditions for the aforementioned polymer. As a result, infrared absorption derived from a phenyl group was observed at 3,600 cm⁻¹, infrared absorption derived from ester bond was observed at 1,730 cm⁻¹, and infrared absorption derived from a phosphorylcholine group was observed at 1,200 to 1,100 cm⁻¹. According to the result of the NMR measurement, the composition of the monomer units in the polymer was as follows: MPC/p-VPB/BMA = 59/6/35 (mole %). The molecular weight was obtained utilizing gel permeation chromatography, and calculation was made utilizing polyethylene oxide as a reference substance. As a result, the number average molecular weight was 12,000.

### [Example 24]

### <Synthesis of polymer (PMBpV) containing a phosphorylcholine group and a phenylboronic acid group simultaneously>

53 g of MPC was weighed and put into a flask, and 300 mL of ethanol was added thereto. With stirring, the inside of the container was subjected to substitution with argon. Next, 8.9 g of *p*-VPB, 8.5 g of BMA and 3.7 g of t-butylperoxyneodecanoate were added thereto, and the mixture was stirred so that it became homogeneous. After the flask was plugged with an airtight stopper, it was heated to 60°C and stirred for 2.5 hours. The obtained solution was taken out therefrom, and the solution was added dropwise to 6,000 mL of a mixed solution of diethylether/chloroform (8/2) to obtain a solid polymer. The yield was about 70%.

This was dried under reduced pressure, thereby obtaining a polymer (PMBpV). This polymer was analyzed according to the IR analysis conditions for the aforementioned polymer. As a result, infrared absorption derived from a phenyl group was observed at 3,600 cm⁻¹, infrared absorption derived from ester bond was observed at 1,730 cm⁻¹, and infrared absorption derived from a phosphorylcholine group was observed at 1,200 to 1,100 cm⁻¹. According to the result of the NMR measurement, the composition of the monomer units in the polymer was as follows: MPC/*p*-VPB/BMA = 54/13/33 (mole %). The molecular weight was obtained utilizing gel permeation chromatography, and calculation was made utilizing polyethylene oxide as a reference substance. As a result, the number average molecular weight was 23,000.

### [Example 25]

### <Synthesis of polymer (PMBpV) containing a phosphorylcholine group and a phenylboronic acid group simultaneously>

14.76 g of MPC was weighed and put into a flask, and 300 mL of ethanol was added thereto. With stirring, the inside of the container was subjected to substitution with argon. Next, 2.96 g of *p*-VPB, 4.27 g of BMA and 3.7 g of t-butylperoxyneodecanoate were added thereto, and the mixture was stirred so that it became homogeneous. After the flask was plugged with an airtight stopper, it was heated to 60°C and stirred for 4 hours. The obtained solution was taken out therefrom, and the solution was added dropwise to 6,000 mL of a mixed solution of diethylether/chloroform (8/2) to obtain a solid polymer. The yield was about 70 %. This was dried under reduced pressure, thereby obtaining a polymer (PMBpV). This polymer was analyzed according to the IR analysis conditions for the aforementioned polymer. As a result, infrared absorption derived from a phenyl group was observed at 3,600 cm⁻¹, infrared absorption derived from ester bond was observed at 1,730 cm⁻¹, and infrared absorption derived from a phosphorylcholine group was observed at 1,200 to 1,100 cm⁻¹. According to the result of the NMR measurement, the composition of the monomer units in the polymer was as follows: MPC/*p*-VPB/BMA = 43/11/46 (mole %).

The molecular weight was obtained utilizing gel permeation chromatography, and calculation was made utilizing polyethylene oxide as a reference substance. As a result, the number average molecular weight was 4,000.

### [Examples 26-38]

### <Preparation of three-dimensional crosslinked matrix>

Each of the polymers obtained in Examples 22 to 25 was dissolved in water to prepare a polymer aqueous solution at a predetermined concentration. Meanwhile, PVA was dissolved in warm water to prepare an aqueous solution, and after that, it was diluted with water to a predetermined concentration. Two types of PVAs, i.e., PVA having a polymerization degree of 400 to 600 (number average molecular weight: 22,000) (abbreviated as PVA500) and PVA1000 were used. By mixing them together at room temperature, three-dimensional crosslinked matrices were prepared. Table 2 shows the results of formation of three-dimensional crosslinked bodies at respective concentrations. In Table 2, the volume mixing ratio between PMBpV and PVA is described as PMBpV/PVA mixing ratio. It is understood from the results in Table 2 that the mixed solution, which is a liquid, became three-dimensional crosslinked matrices and that the solid (gel) state was achieved with the medium included. Further, three-dimensional crosslinked matrices can be produced even when the polymer concentration and the mixed composition are changed. Judgment of production of three-dimensional crosslinked bodies was made using the same criteria as those for Examples 6 to 15. The number average molecular weight of the PMBpVs obtained in Examples 22 to 25 was 4,000 to 23,000, and it was lower than the number average molecular weight of the PMBVs obtained in Examples 1 to 5, which was 39,000 to 81,000. It became clear from the working example that three-dimensional crosslinked matrices can be produced from a low-molecular-weight PMBpV having a number average molecular weight of 4,000 to 23,000.

**Table 2**

| Three-dimensional crosslinked matrices | P M B p V | Concentratio n ( % ) | P V A | P V A concentration ( % ) | PMBpV/ PVA mixing ratio | Judgment |
|---|---|---|---|---|---|---|
| Example 26 | Example 22 | 5 | 1000 | 5 | 1/1 | ○ |
| Example 27 | Example 22 | 5 | 1000 | 2.5 | 1/1 | ○ |
| Example 28 | Example 23 | 5 | 1000 | 5 | 1/1 | ○ |
| Example 29 | Example 23 | 5 | 1000 | 2.5 | 1/1 | × |
| Example 30 | Example 24 | 5 | 1000 | 5 | 1/1 | ○ |
| Example 31 | Example 24 | 5 | 1000 | 2.5 | 1/1 | × |
| Example 32 | Example 25 | 5 | 1000 | 5 | 1/1 | ○ |
| Example 33 | Example 25 | 5 | 1000 | 5 | 1/1 | × |
| Example 34 | Example 22 | 5 | 500 | 5 | 1/1 | ○ |
| Example 35 | Example 22 | 5 | 500 | 5 | 2/1 | ○ |
| Example 36 | Example 22 | 5 | 500 | 5 | 3/1 | ○ |
| Example 37 | Example 24 | 5 | 1000 | 5 | 2/1 | ○ |
| Example 38 | Example 25 | 5 | 1000 | 5 | 2/1 | ○ |

### [Example 39]

### <Study on adhesion in Achilles tendon-damaged rat model>

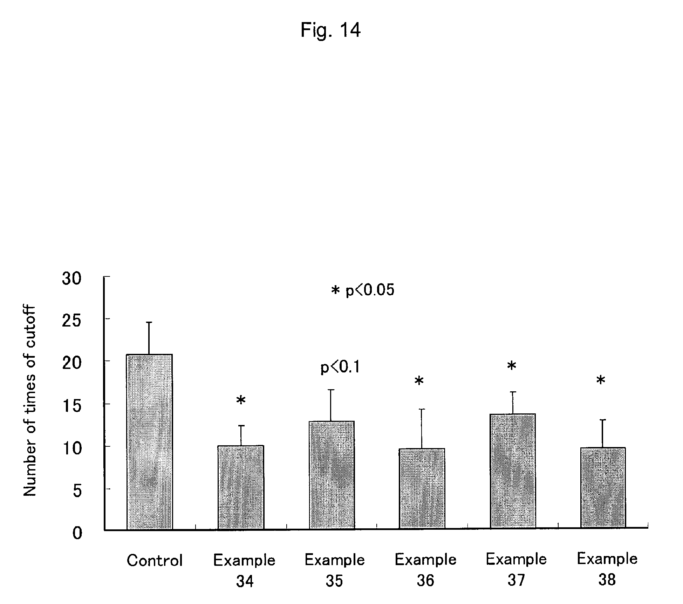

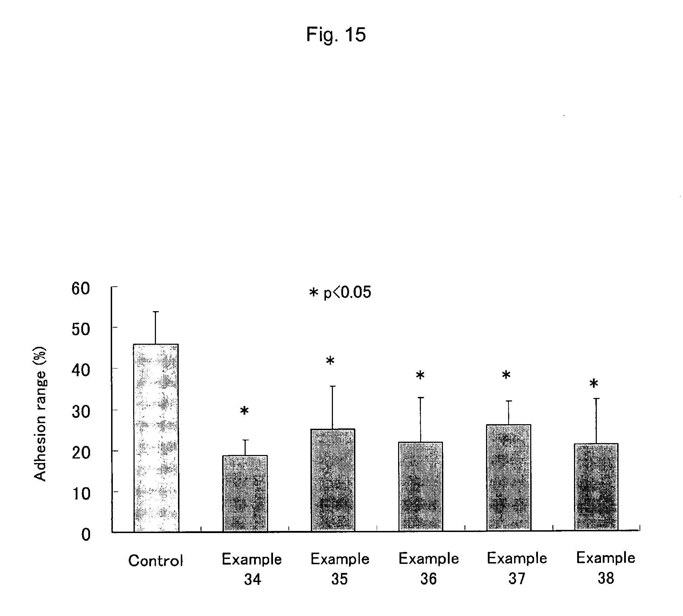

An Achilles tendon of the right foot of a rat was partially cut and sutured, and the three-dimensional crosslinked bodies of Examples 34, 35, 36, 37 and 38 (abbreviated as the BV gels) were adhered to the area surrounding the sutured portion. After the wound was closed, the foot was externally fixed using a plaster cast. Note that in order to limit the movement of the Achilles tendon in the plaster cast, the plantaris tendon that is positioned in parallel to the Achilles tendon was removed. The wound was opened 2 weeks after the surgery, and the degree of adhesion around the sutured portion was evaluated. When the degree of adhesion was evaluated based on the number of fibrous adhesions around the repaired tendon that required sharp dissection, the number of times was obviously decreased in the case of the BV gel group of Examples 34 to 38 compared to the control group 2 weeks after the suture (only distilled water was used) (Figure 14). Further, when evaluation was made based on the ratio of the portion adhered to the surrounding tissue in the circumference of the Achilles tendon (360°) as the adhesion rate (%), it became clear that the adhesion rate was significantly suppressed by any of the BV gels of Examples 34 to 38 (Figure 15). According to the working example, it became clear that adhesion is significantly suppressed even when using a BV gel prepared by mixing with a PMBV having a number average molecular weight of 4,000 to 23,000.

### INDUSTRIAL APPLICABILITY

According to the present invention, by using a polymer having a phosphorylcholine group that has the same structure as that of the cell membrane surface in a molecule, a tissue adhesion prevention material and a joint contracture prevention material comprising, as the main component, a polymeric composition which provides biocompatibility and hydrophilicity can be provided. In addition, according to the present invention, three-dimensional crosslinked matrices can be prepared at ordinary temperature under ordinary pressure in a water system without a chemical or physical technique, and a tissue adhesion and/or joint contracture prevention material comprising a polymeric composition as the main component, which can be prepared at an affected area locally at the time of surgical procedure, can be provided using a convenient and effective method. Moreover, by forming a tissue adhesion prevention material and a joint contracture prevention material by utilizing the above-described polymeric composition and the three-dimensional crosslinked matrices, the problems of the prior art can be easily solved. In view of this, the tissue adhesion and/or joint contracture prevention material of the present invention is remarkably useful.

## Claims

1. A tissue adhesion and/or joint contracture prevention material, which comprises, as the main component, a composition comprising a compound having a polyvalent hydroxyl group and a polymer containing a phosphorylcholine group and a phenylboronic acid group.

2. A tissue adhesion and/or joint contracture prevention material, which consists of a three-dimensional crosslinked matrix formed by a composition comprising a compound having a polyvalent hydroxyl group and a polymer containing a phosphorylcholine group and a phenylboronic acid group.

3. The tissue adhesion and/or joint contracture prevention material according to claim 1, wherein the compound having a polyvalent hydroxyl group is a polymer.

4. The tissue adhesion and/or joint contracture prevention material according to claim 2, wherein the compound having a polyvalent hydroxyl group is a polymer.

5. The tissue adhesion and/or joint contracture prevention material according to claim 1 or 2, wherein the polymer containing a phosphorylcholine group and a phenylboronic acid group is represented by the following general formula (1): 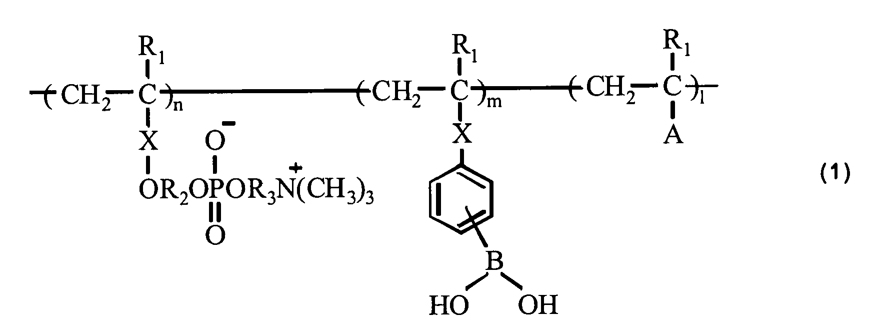 wherein: R₁ represents a hydrogen atom, a methyl group or an ethyl group; R₂ represents an alkyl group having 2 to 12 carbon atoms or an oxyethylene group; R₃ represents an alkyl group having 2 to 4 carbon atoms; X represents a single bond, a substituted or unsubstituted phenyl group, or a group represented by -C(O)-, -C(O)O-, -O-, -C(O)NH-or -S-; A represents a hydrogen atom, a halogen atom or any organic substituent; and n,
m and 1 respectively represent 0.01 to 0.99, 0.01 to 0.99, and 0 to 0.98 (with proviso that the sum of n, m and I is 1.00).

6. The tissue adhesion and/or joint contracture prevention material according to claim 1 or 2, wherein the compound having a polyvalent hydroxyl group is at least one selected from the group consisting of natural saccharides, synthetic saccharides and organic alcohols.

7. The tissue adhesion and/or joint contracture prevention material according to claim 1 or 2, wherein the compound having a polyvalent hydroxyl group is at least one selected from the group consisting of polysaccharides and synthetic polymeric alcohols.
